# EUROPÄISCHE PATENTANMELDUNG

(11) **EP 1 839 652 A1**
(43) Veröffentlichungstag der Anmeldung: **03.10.2007**
(21) Anmeldenummer: 07005118.0
(22) Anmeldetag: 13.03.2007
(51) Int. Cl.: A61K 31/045, A61K 31/19, A61K 35/00, A61P 25/28

(54) **Mittel zur positiven Beeinflussung der Alzheimer Krankheit und/oder zur Alzheimerprophylaxe**

(30) Priorität: 21.03.2006 DE 102006012876
(71) Anmelder: CLARIANT INTERNATIONAL LTD., 4132 Muttenz (CH)
(72) Erfinder: Kammermeier, Bernhard, Dr., 80687 München (DE); Heinrichs, Franz-Leo, Dr., 86456 Gablingen (DE); Krendlinger, Ernst, Dr., 86316 Friedberg (DE); Neumaier, Manfred, 86356 Neusäss (DE)
(74) Vertreter: Paczkowski, Marcus

(57) **Zusammenfassung**

Die Erfindung betrifft ein Mittel zur Alzheimerprophylaxe und/oder zur therapeutischen Behandlung bereits bestehender Symptome vom Typus der Alzheimer Krankheit enthaltend eine Mischung aus VLCFA3, insbesondere auf Basis von Montanwachsen und/oder deren Derivaten oder von Guerbetsäuren oder den entsprechenden Komponenten von Carnaubawachs, Shellack oder Policosanolen oder VLCFA3 aus Naturwachsen, ggf. in Kombination mit physiologisch verträglichen Kupfersalzen wie Kupferorotat und in Kombination mit DMAE. Das Mittel kann erfindungsgemäß als Speisezusatz oder als Getränk oral appliziert werden.

## Beschreibung

Die vorliegende Erfindung betrifft ein Mittel enthaltend langkettige Carbonsäuren oder langkettige Alkohole auf Basis von Rohmontanwachsen und/oder deren Derivaten, und/oder Guerbetsäuren oder Guerbetalkohole oder weitere Komponenten wie Rohcarnaubawachs oder Shellack, ggf. in Kombination mit physiologisch verträglichen Kupfersalzen und Dimethylaminoethanol, und seine Verwendung zur positiven Beeinflussung der Alzheimer Krankheit oder damit assoziierter degenerativer Krankheitsbilder und/oder zur Alzheimerprophylaxe.

Als langkettig werden definitionsgemäß gesättigte oder partiell ungesättigte Kohlenwasserstoffreste mit 14 bis 40 C-Atomen, vorzugsweise mit 28 bis 40 C-Atomen, angesehen. Erfindungsgemäß werden die genannten Mittel vorzugsweise oral appliziert.

Die Alzheimer Krankheit (Morbus Alzheimer) ist die häufigste Ursache einer Demenzerkrankung im Alter. Sie zeichnet sich aus durch erworbene Gedächtnisstörungen in Kombination mit anderen kognitiven Einbußen wie Sprachstörungen, Aufmerksamkeits- und Abstraktionsprobleme, fehlende räumliche und zeitliche Orientierung usw. (S.Teipel, H. Hampel, Bayrisches Ärzteblatt 9/2005, 552-556). Derzeit leiden in Deutschland schätzungsweise 800 000 Menschen an einer leichten bis mittelmäßigen Ausprägung der Alzheimer Krankheit, wobei sich die Zahl der Patienten als Folge der demographischen Entwicklung in den kommenden 20 bis 30 Jahren voraussichtlich verdoppeln wird. Physiologisch weisen die Gehirne von an Morbus Alzheimer erkrankten Personen abnorme clusterartige Ablagerungen auf, sog. Amyloide Plaques aus β-Amyloid im Extrazellulärraum und neurofibrilläre Bündel aus 'tau-Protein' im Intrazellulärraum, die für den Verlust von Gehirnsubstanz verantwortlich gemacht werden (P. H. St George-Hyslop, Spektrum d. Wissenschaft, 3/2002, 44-51).

lm Jahr 2005 wurde die physiologische Funktion des Peptides entschlüsselt dessen Akkumulation im Gehirn zu den Alzheimersymptomen führt (Online Ausgabe, Nature Cell Biology, 2005; doi: 10.1038/ncb 1313; zitiert in: Deutsches Ärzteblatt, Jg. 102, Heft 42, 21.10.2005). Danach spielt das β-Amyloid eine wichtige Rolle im Cholesterinstoffwechsel. Es greift an zentraler Stelle in den Cholesterinstoffwechsel der Nervenzelle ein, die wiederum in der Lage ist, selbst Cholesterin zu produzieren. Zusätzlich aktiviert β-Amyloid die Sphingomyelinasen, die für den Abbau von Sphingomyelin verantwortlich sind. Ein zu hoher Lipidgehalt steigert in den Zellen die Produktion von β-Amyloid und führt damit zu der Gefahr der für die Alzheimer Krankheit typischen Verklumpungen. Bereits früher war ein statistischer Zusammenhang zwischen Lipidprofil/Cholesterinspiegel und altersabhängigem Risiko für die Ausprägung einer Demenz vom Typus der Alzheimer Krankheit bekannt geworden. Es wurde daher bereits vermutet, dass eine Therapie mit Statinen diese Amyloidakkumulation verringern oder sogar ganz verhindern kann.

Während noch 1994 über einen Zusammenhang zwischen einer Verschlechterung einer Alzheimer Krankheit und der supplementiven Gabe von Zink berichtet wurde (A.I. Bush et al., Science Vol.265, 02.09.1994, 1464-1467), wurde 2001 über den in vitro inhibierenden Effekt von Kupfer auf die β-Amyloidaggregation berichtet (J. Zou et al., Angewandte Chemie 2001, 113, Nr. 12, 2334-2337). Damit war die Frage aufgeworfen worden, ob Kupfermangel mit Morbus Alzheimer assoziiert ist und ob eine therapeutische Kupfergabe möglicherweise ein Behandlungsansatz bei der Alzheimer Krankheit sein könnte. In einer Studie an APP transgenen Mäusen konnte gezeigt werden, dass die β-Amyloidspiegel durch orale Verabreichung von Kupfer reduziert wurden (http://www.meb.uni-bonn.de/psychiatrielnewpage/kupfer.htm).
An der Universität des Saarlandes in Homburg wurde eine Kupferstudie bei Alzheimer Patienten gestartet mit bis dato Erfolg versprechenden Resultaten (http://idw-online.de/pages/de/news112978). Eine weitere medikamentöse Säule in der Behandlung von Demenzen vom Typus der Alzheimer Krankheit sind schließlich Cholinesterasehemmer, weil der Verlust der kognitiven Funktion u.a. durch zentrale cholinerge Defizite zustande kommt. Eine Vorstufe von Cholin, das Dimethylaminoethanol (DMAE), ist in kleinen Konzentration stets im Gehirn präsent; ihm wird eine nootrope Funktion zugeschrieben
(http://vitabasix.com/de/media/documents/171.pdf?PHPSESSID=05877eb1d1b7c344 a128c4bb3339bc0a).

In einem toxikologischen Review (R. Tice, Intergrated Laboratory Systems, P.O.B. 13501, Research Triangle Park, North Carolina 27709, June 1997) wird über milde mentale Stimulation bei Versuchspersonen berichtet, die orale Verabreichungen des Tartratsalzes von DMAE in Dosen von 10 bis 20 mg erhalten hatten.

Es ist auch bekannt, dass aliphatische langkettige Alkohole etwa aus dem Wachs von Naturprodukten (E. Ernst, MMW Nr. 23/2002, Seite 20) zu einer Reduzierung der Cholesterinwerte führen. In der DE 10 2004 055 858 wird hingegen vorgeschlagen, die Alkoholkomponente synthetischer Wachse oder verzweigte langkettige synthetisch zugängliche Alkohole wie Montanwachsalkohole, Guerbetalkohole zu verwenden und damit eine Erhöhung der HDL-Konzentration und eine Erniedrigung der LDL-Konzentration herbeizuführen. Vergleichsstudien zeigten eine zu herkömmlichen Statinen (= Simvastatin und Pravastatin) äquivalente Wirkung bei fehlendem Nebenwirkungsprofil im Dreijahresvergleich. Darüber hinaus wird ein Interkonversionsmechanismus im endoplasmatischen Retikulum postuliert, in dem sehr langkettige aliphatische Carbonsäuren (VLCFA = very long chain fatty acids) in einem Fett-Alkohol-Zyklus in die analogen langkettigen Alkohole bzw. Aldehyde reversibel konvertiert werden. Das Bedeutet, dass alle drei Komponenten: Säuren, Alkohole, Aldehyde, abgekürzt als VLCFA3 = very long chain fatty acid plus alcohol plus aldehyd, jeweils ineinander umwandelbar sind, wobei enzymatisch u.a. körpereigene Lipasen, Fettsäuretransportproteine, Aldehyddehydrogenasen, Alkoholdehydrogenasen, CoA-Ligasen u.a. an dieser Umwandlung beteiligt sind (J.L.Hargrove et al., Exp. Biol. Med. 229, 215-226, 2004 und darin zitierte Literatur). Daraus wird abgeleitet, dass langkettige Alkohole in ihrer Substanzeigenschaft bezüglich des oben beschriebenen Wirkmechanismus durch die analogen langkettigen Carbonsäuren bzw. Aldehyde unter Erhalt des Wirkprinzips substituiert werden können.

Eine Kombination aus allen oben genannten Wirkprinzipien, (1) VLCFA3 zur Normalisierung des Lipidprofils mit der Folge der Verringerung der neuronalen β-Amyloidproduktion, (2) physiologisch verträgliche Kupfersalze zur Inhibierung der Amyloidaggregation und (3) DMAE, das die Blut-Hirn-Schranke besser überwindet als Cholin, als physiologischen Precursor für Cholin und damit Acetylcholin, greift unter Zugrundelegung der vorgenannten Studienergebnissen an voneinander unabhängigen Endpunkten des pathologischen Geschehens der Alzheimer Krankheit ein und ermöglicht zudem die Vermeidung von rezeptpflichtigen Statinen.

Gegenstand der vorliegenden Erfindung ist daher ein Mittel zur Alzheimerprophylaxe und/oder zur therapeutischen Behandlung bereits bestehender Symptome vom Typus der Alzheimer Krankheit enthaltend mindestens eine Komponente aus VLCFA3, insbesondere auf Basis von Montanwachsen oder deren Derivaten, und/oder Guerbetsäuren oder Guerbetalkoholen oder den entsprechenden Komponenten von Carnaubawachs oder Shellack oder Policosanolen, dessen kennzeichnende Merkmale darin zu sehen sind, dass die Kettenlänge der Kohlenwasserstoffreste in der Komponente VLCFA3 im Bereich von 14 bis 40 C-Atomen liegt, vorzugsweise von 28 bis 40 C-Atomen, dass die Anzahl der C-Atome der Kohlenwasserstoffreste vorzugsweise eine gerade Zahl ist und dass die Carbonsäuren ggf. Mengen im Bereich von 0 bis 10 Mol-% an unverzweigten Dicarbonsäuren enthalten.

Das erfindungsgemäße Mittel kann zusätzlich als weitere Komponente physiologisch verträgliche Kupfersalze wie Kupferorotat enthalten. Diese entfalten zusammen mit der Komponente VLCFA3 insofern synergistische Effekte, als sie an jeweils unterschiedlichen Eckpunkten des pathopysiologischen Geschehens bei der Erkrankung angreifen. Alternativ dazu kann das erfindungsgemäße Mittel neben der Komponente VLCFA3 auch noch DMAE (Dimethylaminoethanol) enthalten. Auch diese Kombination kann zur Ausbildung von synergistischen Effekten führen. In einer besonderen Ausführungsform der Erfindung enthält das Mittel die Komponente VLCFA3 in Kombination mit physiologisch verträglichen Kupfersalzen wie Kupferorotat und in Kombination mit DMAE. Durch die Dreierkombination lassen sich die synergistischen Effekte bei der Wirkung des Mittels noch weiter steigern.

Das erfindungsgemäße Mittel wird in einer täglichen Dosis entsprechend 1 bis 1000 mg, bevorzugt von 5 bis 500 mg, besonders bevorzugt von 5 bis 100 mg, appliziert, bezogen auf die Komponente VLCFA3, während die Komponente Kupfersalz in einer täglichen Dosis von 0 bis 10 mg, vorzugsweise von 1 bis 10 mg, bezogen auf das Gewicht an Kupfer, und DMAE in einer täglichen Dosis von 0 bis 200 mg, vorzugsweise von 1 bis 170 mg, bezogen auf das Gewicht an DMAE eingesetzt wird.

Bevorzugt werden die VLCFA dieser langkettigen Wachsester auf Basis von Montanwachsen, sowie deren Derivaten (VLCFA3) und Guerbetderivate oder den entsprechenden Komponenten von Carnaubawachs, oder Shellack durch Verseifungsreaktionen, oder durch Oxidation mit Chromschwefelsäure (in der Literatur als Gersthofen Prozess bekannt) aus Wachsen erhalten oder sie werden durch Anwendung der aus der Literatur bekannten Guerbetreaktion gewonnen. Die genannten VLCFA aus Montanwachsen oder Guerbetderivaten sind als langkettige unverzweigte oder verzweigte Säuren in gewerbetoxikologischen Studien zusätzlich als untoxisch erkannt worden (siehe dazu: Aufsatz "Montan Wax" in Ullmann's Encyclopedia of Industrial Chemistry, Vol A 28, 5.Auflage, Weinheim, Verlag Chemie, S.122-126).

## Patentansprüche

1. Mittel hauptsächlich zur Alzheimerprophylaxe aber auch zur therapeutischen Behandlung bereits bestehender Symptome vom Typus der Alzheimer Krankheit enthaltend wenigstens eine Komponente aus VLCFA3, insbesondere auf Basis von Montanwachsen oder deren Derivaten, und/oder Guerbetsäuren oder Guerbetalkoholen oder den entsprechenden Komponenten von Carnaubawachs oder Shellack oder Policosanolen, **dadurch gekennzeichnet, dass** die Kettenlänge der Kohlenwasserstoffreste in der Komponente VLCFA3 im Bereich von 14 bis 40 C-Atomen liegt, vorzugsweise von 28 bis 40 C-Atomen, dass die Anzahl der C-Atome in den Kohlenwasserstoffresten vorzugsweise eine gerade Zahl ist und dass die Carbonsäuren ggf. Mengen im Bereich von 0 bis 10 Mol-% an unverzweigten Dicarbonsäuren enthalten.

2. Mittel nach Anspruch 1, **dadurch gekennzeichnet, dass** es in der Komponente VLCFA3 mindestens VLCFA auf Basis von Montanwachsen enthält oder deren Derivaten oder von Guerbetsäuren oder den entsprechenden Komponenten von Carnaubawachs oder Shellack oder VLCFA aus Naturwachsen.

3. Mittel nach Anspruch 1 oder 2, **dadurch gekennzeichnet, dass** es zusätzlich zu der Komponente VLCFA3 noch physiologisch verträgliche Kupfersalze wie Kupferorotat enthält, wobei die vorgenannten Komponenten (Kupfersalze und VLCFA3) in Kombination synergistische Wirkung entfalten indem sie an jeweils unterschiedlichen Eckpunkten des pathophysiologischen Geschehens eingreifen.

4. Mittel nach Anspruch 1 oder 2, **dadurch gekennzeichnet, dass** es zusätzlich zu der Komponente VLCFA3 noch Dimethylaminoethanol (DMAE) enthält, wobei auch eine synergistische Wirkung entfaltet wird.

5. Mittel nach einem oder nach mehreren der Ansprüche 1 bis 4, **dadurch gekennzeichnet, dass** es zusätzlich zu der Komponente VLCFA3 noch physiologisch verträgliche Kupfersalze wie Kupferorotat und in Kombination damit auch DMAE enthält, mit den in Anspruch 3 und 4 genannten synergistischen Effekten.

6. Verwendung eines Mittels nach einem oder nach mehreren der Ansprüche 1 bis 5, **dadurch gekennzeichnet, dass** es in einer täglichen Dosis von 1 bis 1000 mg, vorzugsweise von 5 bis 500 mg, besonders bevorzugt von 5 bis 100 mg, bezogen auf die Menge an Komponente VLCFA3 eingesetzt wird.

7. Verwendung eines Mittels nach einem oder nach mehreren der Ansprüche 1 bis 5, **dadurch gekennzeichnet, dass** es in einer täglichen Dosis von 0 bis 10 mg, vorzugsweise von 1 bis 10 mg, bezogen auf das Gewicht an Kupfer, eingesetzt wird und dass es in einer Menge von 0 bis 200 mg, vorzugsweise von 1 bis 170 mg, bezogen auf die Menge an DMAE eingesetzt wird.

8. Verwendung eines Mittels nach einem oder nach mehreren der Ansprüche 1 bis 5 als Speiseadditiv oder in emulgierter Form als Getränk.
